# EUROPEAN PATENT SPECIFICATION

(11) **EP 2 678 436 B1**
(45) Date of publication and mention of the grant of the patent: **04.11.2020**
(21) Application number: 11859141.1
(22) Date of filing: 24.02.2011
(51) Int. Cl.: C12P 7/06, C12N 1/16, A01N 37/16

(54) **METHOD FOR PREVENTING BACTERIAL INFECTION IN A FERMENTATION PROCESS**
VERFAHREN ZUR PRÄVENTION VON BAKTERIELLEN INFEKTIONEN IN EINEM FERMENTATIONSPROZESS
PROCÉDÉ DE PRÉVENTION D'INFECTION BACTÉRIENNE DANS UN PROCÉDÉ DE FERMENTATION

(43) Date of publication of application: 01.01.2014
(73) Proprietor: Kemira OYJ, 00100 Helsinki (FI)
(72) Inventor: MOREIRA DA COSTA. Marcelo, 06543-001 Santana de Parnaíba -SP (BR); SENRA, Vanderlei, 06519-480 Santana de Parnaíba - SP (BR)
(74) Representative: AWA Sweden AB
(86) International application number: PCT/BR2011/000050
(87) International publication number: WO 2012/113042

(56) References cited:
- WO-A1-94/20424
- WO-A1-2007/145858
- WO-A1-2010/093765
- WO-A2-2007/149450
- CA-A1- 2 300 807
- US-A- 1 727 223
- US-A1- 2009 104 157
- US-A1- 2010 143 307
- US-A1- 2010 297 719

## Description

The present invention relates to an improved method for preventing bacterial infection in a fermentation process by using performic acid.

Ethanol can be produced from almost any material which either exists in the form of, or can be converted into, a fermentable sugar. There are many sources of natural sugars available for fermentation, such as sugar beets and sugar cane, but any kind of carbohydrates such as starch and cellulose can be also be converted into fermentable sugars which then ferment into ethanol. Even today, throughout most of the world, ethanol is produced through the fermentation process.

If a carbohydrate such as starch or cellulose is used for the fermentation, then a saccharification or sugar-producing step (process of breaking a complex carbohydrate, such as starch or cellulose, into its monosaccharide components) is required before the fermentation, to have the carbohydrate converted into a fermentable sugar. This saccharification step is well-known for the man skilled in the art and does not constitute a part of the present invention. After the saccharification step is completed, yeast is added to the obtained fermentable sugars and then fermentation begins. Alternatively, today many distillers add the saccharification enzyme to the fermenter with the yeast. This simultaneous saccharification and fermentation allows for higher concentrations of starch to be fermented.

Of course, if the sugar source comes from crops such as sugar cane, sugar beets, fruit or molasses, saccharification is not necessary and fermentation can begin straight away with the addition of yeast and water.

The sugar-containing material from sugar cane can be obtained for example by the following process: Once harvested, sugarcane is usually transported to the plant by semitrailer trucks. After a quality control, the sugarcane may be washed. Then it is chopped, and shredded by revolving knives. Sugar is produced by first squeezing the juice out of the stems. The raw juice is clarified, impurities and solids are removed, and then it is thickened. After this follows a series of crystallization steps to produce sugar crystals, which are removed. The remaining syrup is molasses. Fermentation of sucrose from sugar cane can be conducted using the juice directly obtained from squeezing the sugar cane stalks, or from the molasses.

Typical sugar-containing material that is used as a starting material for fermentation is made from cane juice and/or molasse and water to obtain a sugar concentration of 18-23° Brix and it is often called a must.

In the fermentation process, yeast is added to a solution of simple sugars. Yeast is a small microorganism which uses the sugar in the solution as source of energy, and in doing so, expels ethanol and carbon dioxide as byproducts. The carbon dioxide comes off as a gas, bubbling up through the liquid, and the ethanol stays in solution. Unfortunately, the yeast stagnates when the concentration of the ethanol in solution approaches about 18 percent by volume, whether or not there are still fermentable sugars present.

The fermentation takes place in one or several fermentation vessels. In this Fermentation process, sugars are transformed into ethanol by addition of yeast. Fermentation time varies from four to twelve hours or more, resulting in an ethanol content of 7-10% by total volume (°GL), called fermented wine. The yeast is recovered as yeast cream from this wine, normally by means of a centrifuge. Making use of the different boiling points, the ethanol in the fermented wine is separated from the main remaining solid components.

In order to produce large quantities of ethanol, the common practice has been to use a batch process wherein extremely large fermentation vessels capable of holding up to 600 m³ to 4000 m³.

After fermentation, distillation processes are used to remove the ethanol from the fermentation solution and to further concentrate it. Distillation towers capable of such separations and concentrations are well-known in the art.

The fermentation step is one of the most important steps in ethanol production. Furthermore, one of the main concerns with conventional fermentation systems is the difficulty of maintaining acceptable low levels of bacteria in the large-sized batches and with the long fermentation period. Unfortunately, the optimum atmosphere for fermentation is also extremely conducive to bacterial growth. Should a batch become infected, not only must the yeast and sugar solution be discarded, but the entire fermentation vessel must be emptied, cleaned, and sterilized. Such an occurrence is both time-consuming and costly.

Additionally, many of these bacteria compete with the yeast for sugar, thereby reducing the amount of ethanol that is produced, thereby reducing the fermentation yield. Bacteria can grow nearly ten times faster than yeast, thus contamination in these areas are inevitable. Upon the consumption of sugar, these bacteria produce lactic acid and other undesired byproducts.

Further, if the fermentation vessels are not properly disinfected or sterilized between batches or uses, bacteria and other undesirable microorganisms can become attached to the interior walls of the fermentation vats where they will grow and flourish and form biofilm. These undesirable microorganisms may consume valuable quantities of the carbohydrates, or sugars, thus reducing the production of ethanol, or they may contaminate ethanol co-products such as animal feed. The economics and efficiency of fermentation processes are frequently such that they cannot tolerate any such loss of production.

Current methods used to kill these unwanted microorganisms, among others, often involve introduction of exogenous agents, such as antibiotics, quaternary ammonium compounds and chlorine dioxide to the fermentation before or during production of ethanol.

Commonly, synthetic chemical antibiotics are added to the fermentation vessels in an attempt to decrease the growth of lactic acid producing bacteria. Antibiotics are expensive and can add greatly to the costs of a large- scale production.

There is much to be desired in the field of ethanol production for achieving effective means for a bacterial infection prevention that is safe, low cost, and environmentally sound, yet which enhances, rather than degrades or limits, efficient alcohol producing microorganism activity. There is a need in the art for a compound and a method which increase fuel ethanol yields from fermentation. Or in other terms, there is a need in the art for a compound and a method to reduce the population of unwanted microorganisms in fuel ethanol fermentation in order to increase ethanol yield. Several additives have been suggested with more or less success. Thus, in WO 2007/149 450 a process for preventing bacterial growth in fermentation processes is disclosed, according to which a stabilized chlorine dioxide is added. However, chlorine dioxide treatment requires a low pH of less than 45.In US 1 727 223 a process is described for improving the fermentation process of a sacchariferous material, according to which an inorganic or organic peroxide is added to said material. Moreover, in WO 2004/072291 the use of hop acids is disclosed in fuel ethanol production.

In view of the above, it is a purpose of the present invention to improve the yield of fermentation processes and in particular the fermentation of a sugar-containing material to ethanol.

A further object of the present invention is to minimize or even eliminate the use of sulphuric acid and antibiotics.

An additional benefit of this invention is that the excess yeast can be easily utilized in the fodder industry because it does not contain residues from the antibiotics and biocides.

According to the present invention, a better disinfection in a fermentation process has been achieved in a surprisingly easy and economical way by adding performic acid to the material to be, or being, fermented.

### DETAILED DESCRIPTION OF THE INVENTION

As mentioned above, in order to reduce the population of undesired micro-organisms and/or bacteria in the fermentation process of sugar-containing material, a disinfectant comprising performic acid is added.
The present invention is defined in the appended claims.
Performic acid is a colourless liquid which is miscible with water and ethanol. Solutions of performic acid are unstable as such so they are normally produced as equilibrium solution on the place of use. They are produced as aqueous solutions by reaction of formic acid and hydrogen peroxide in the presence of a catalyst, the catalyst being for example a mineral acid selected from sulphuric acid, phosphoric acid hydrochloric acid and nitric acid. The catalyst may also be a compound containing at least one ester group and/or group differing from a carboxylic acid group and an alcoholic group, preferably a carboxylic acid ester, thereby forming aqueous equilibrium solutions comprising performic acid.

The performic acid may be prepared from formic acid and hydrogen peroxide. The molar ratio of formic acid and hydrogen peroxide may be from 1:10 to 10:1, preferably 1:3 to 3:1. A typical mixture of performic acid and the starting materials, which mixture is an equilibrium solution and may be used as a disinfectant according to the invention, contains approximately 5 - 30 % by weight of formic acid, approximately 10 - 40 % by weight of hydrogen peroxide and approximately 3 -25 % by weight, preferably 5 - 20% by weight of performic acid. For the purpose of the preparation of the performic acid, technical-grade formic acid of a concentration of for example 85%by weight is typically employed. Hydrogen peroxide is employed preferably in a concentration of 20-50% by weight, more preferably about 35 - 50% by weight. Higher concentrations pose the risk of an explosion. If a mineral acid is used as a catalyst, the amount of acid catalyst, for example the amount of sulphuric acid in the equilibrium solution, is approximately 1 - 15 % by weight. There is also water in the equilibrium solution.

In the process of the present invention, performic acid is added to one or more of the fermentable sugar-containing materials such as to the molasses and fermentable juice, to the inoculant (the yeast), or to the fermentation system, in an amount effective to reduce the bacteria population and thus prevent the formation of undesired by-products like acetic acid or lactic acid in the system. That is, performic acid is added prior to substantial growth of bacteria in the fermentation system, such as prior to the introduction of any or all of the ingredients necessary to initiate the fermentation process The need for adding performic acid can be determined by measuring concentrations of acetic acid and lactic acid in the system or by other suitable means.

A disinfectant comprising performic acid may be added before and/or during the fermentation process. Performic acid may be added to the sugar-containing material or to the inoculant (yeast cream) prior to their introduction into the fermentation system. It may for instance be added in the form of the above mentioned equilibrium solution. In one embodiment, performic acid is added to the fermentation vessel(s) together with the flow stream of a sugar-containing material to be fermented, comprising molasseand/or sugar-containing juice. In another embodiment, performic acid is added to the yeast. In still another embodiment, performic acid is added to the fermentation vessel(s) together with the flow stream of a sugar-containing material to be fermented, such as molasse and/or sugar-containing juice, and to the yeast. The fermentation process may be either batch or continuous. By the term "fermentation system" is meant the batch or continuous flow liquefaction train and fermentation tanks, vessels, reactors, heat exchangers, pipings (such as a plug flow reactor) or combinations thereof, in which the fermentation of sugar occurs. In the present patent application, the term "fermentation vessel" is used to any type of holder for the fermentation process, such as a tank, a reactor of any kind, a cistern, a hopper, a vat, etc. Alternatively or in addition thereto, performic acid may be added as a separate stream to the fermentation system, apart from the fermentable sugar and inoculant. In a batch process, the performic acid may alternatively be added before, during and/or following the addition of the fermentable sugar and/or inoculant to the fermentation vessel. When the inoculant is yeast, performic acid may also be added to the yeast propagation tank. However, basically, the performic acid should be added prior to substantial growth of bacteria in the fermentation system. Further, the performic acid may be added to the fermentation vessel simultaneously or after the addition of sugar-containing material to be fermented. Moreover, as mentioned above, the performic acid may be added to the fermentation vessel(s) together with the inoculant, for instance yeast.

Performic acid is added in an effective amount, and by "effective amount" is meant an amount that is capable to reduce the bacteria population without adversely affecting the fermentation process. Such conditions allow the inoculant to quickly and effectively convert the fermentable sugar to ethanol. Performic acid is added in an amount effective to substantially reduce the bacteria population but has little impact to the yeast or on the major variables in the fermentation process. This amount will typically be from about lto 2000mg of performic acid per liter of sugar-containing material in the fermentation vessel(s) when all of the reactants have been added to the system. It will be understood that the amount of performic acid needed will depend on the total bacteria load introduced to the system. Additional factors to consider in determining the amount of performic acid to add include timing of inoculant (yeast) addition and pH, and to some extent also where in the system the performic acid is added. Preferably, the amount of performic acid added shall result in 2-400 , more preferably 5- 100 mg of performic acid per liter of sugar-containing material in the fermentation vessel(s). This amount is substantial enough to minimize process interruptions due to bacterial contamination, and to minimize the need for other biocides; antibiotics. Preferably, the sugar-containing material in the fermentation system has a pH of 3-8, preferably 4-8 and most preferably 5,5-8.

By operating a fermentation plant in accordance with this invention, a reduced rate in frequency of (even with potential elimination of) deleterious effects of bacterial infection, is achieved. Thus, in the process of this invention, long term productivity and profitability increase in the operation of a fermentation plant.

In the process of this invention, fermentation occurs in a batch or continuous fermentation system. The product mixture from the fermentation system comprises mainly ethanol, water, and inoculant such as yeast. After discharge from the fermentation system, conventional process steps for separation and purification or other processing of the ethanol may be performed. The fermentation product may be distilled to separate the ethanol from the bulk of the water present and from the solids. The solids may be recovered. The distilled ethanol may be further treated, for example by contacting it with molecular sieves, to remove remaining water. In beverage production, aging, blending or other processing may be required. Purified fuel ethanol may be treated with a denaturing agent. Co-produced carbon dioxide and solids can also be recovered.

In the process for reduction of the population of unwanted bacteria, the aqueous performic acid solution can be added to the process medium continuously, discontinuously or using shock dosage. The solution may be added to the process medium through closed dosing systems. That means that control of micro-organisms may be done under the use of the process installations (closed dosing systems) already available.

Generally, the temperature of the sugar-containing material in the fermentation system to be treated is below 100°C, in one aspect below 50°C and in another aspect below 40°C.

The process for reducing the population of bacteria and other unwanted micro-organisms can be automated by the use of time controls for the dosing pumps and valves. Also in this case, the efficiency increases. The improved effect means that the overall concentration of active ingredients can be reduced, which produces a number of advantages. Either reduced costs are achieved through lower dosing or the same dosing produces a better effect.

In the following example, performic acid was prepared by mixing first110g of 85 w-% formic acid, 24 g of water and 26g of concentrated sulphuric acid. Then the resulting solution was mixed with 192g of 50w-% hydrogen peroxide, to generate performic acid as an equilibrium solution. The performic acid concentration was calculated to be 99 % by weight.

Total viable bacteria and viable yeasts in the samples herein were measured as colony forming units (CFU) per unit of volume (i.e., CFU/ml). The viability of yeasts and their ability to reproduce were measured by using a microscopical method, where specific dye (erythrosine) was used to visualize the amount and form (live, dead, budding) of yeast cells.

### Example 1.

The yeast cream samples (recovered from the wine, through a centrifuge) were diluted with lake water in a ratio of 30 % + 70 %, respectively. The diluted yeast cream samples (later on called yeast cream solutions) were exposed to varying concentrations of performic acid in the equilibrium solutionas follows: 49,5 ppm, 74,3 ppm and 99 ppm. In a control sample, no performic acid was added to the yeast cream solution. The effect of performic acid on bacteria was determined by one method (method 1), and on yeast cells by two methods (methods 1 and 2).

### Method 1.

The exposed samples and the control sample were held in +30°C for 30 minutes, after which the amount of survived microbes was enumerated. The method used was a Serial dilution and cultivation method, where the effect of performic acid on the yeast cream solution was measured via dilution and cultivation of the sample after the exposure, to enumerate the amount of microbial growth. This method entail the dilution of each sample by a factor large enough to enable clear separation of individual microbial colonies on Petri™ films (from 3M); thus allowing the colonies to be individually counted. Where diluted samples had been applied and inoculated in, the Petri™ films were incubated at +35 °C for 48 hours (bacteria) or at +30 °C for 48 hours (yeasts). Results are provided in Table 1.

### Method 2.

The exposed samples and the control sample were held in +30°C for 5 hours, after which the specific parameters of yeast cells were measured. These parameters included the amount of living yeast cells, dead yeast cells and budding yeast cells (i.e. reproducing yeast cells). The method used was a standard method, commonly adopted by Brazilian Sugar Cane Industry mills. In the method, a Neubauer chamber was filled with approximately 10 µl of the exposed sample, where the erythrosine dye had been mixed in. On the cover slip, a drop of immersion oil was added, and the above mentioned yeast cell parameters were evaluated by using an immersion objective (100x) of the microscope. The cells that presented pink coloration were counted as dead cells, and uncolored cells as living cells. The budding cells were determined on the basis of the shape of the yeast cell.

The total amount of the yeast cells was counted and compared to the result obtained with Method 1. Results are provided in Table 1 (Neubauer count of yeast cells).
The cell viability indicates the percentage of viable cells over total cells (live + dead). The cell reproductive % indicates the percentage of budding cells over total living cells. The yeast cell viability % and reproductive % are shown in Table 2.

**Table 1.The amount of bacteria and yeasts after the exposure to performic acid.**

| **Concentration of performic acid in Yeast Cream solution** | **CFU/ml Bacteria 48 h, MIC** | **CFU/ml Yeasts 48 h MIC** | **CFU/ml Yeasts 48 h, Neubauer count** |
|---|---|---|---|
| Control, 0 ppm | 1 500 000 | 1 000 000 000 | 420 000 000 |
| 495ppm | 60 | 1 000 000 000 | 380 000 000 |
| 743ppm | 70 | 6 100 000 000 | 400 000 000 |
| 99ppm | 30 | 1 000 000 000 | 640 000 000 |

**Table 2. The yeast cell viability % and reproductive %**

| **Concentration of performic acid in Yeast Cream solution** | **Yeast cell viability %** | **Reproductive yeast cells %** |
|---|---|---|
| Control, 0 ppm | 68,4 % | 6,8 % |
| 495ppm | 71,1 % | 6,8 % |
| 743ppm | 72,1 % | 8,9 % |
| 99ppm | 72,3 % | 4,1 % |

The present invention has been disclosed with particular reference to some specific embodiments thereof, but it should be understood that modifications and changes may be made by the person skilled in the art without departing from the scope of the invention as defined in the appended claims.

## Claims

1. A method for preventing bacterial infection in a process for fermenting a sugar-containing material, **characterized in that** a disinfectant comprising performic acid is used.

2. The method according to claim 1, including using a fermentation system comprising the sugar-containing material and an inoculant such as yeast, wherein the disinfectant is added to the sugar-containing material and/or to the inoculant.

3. The method according to claim 2, **characterized in that** the disinfectant comprises performic acid in the form of an aqueous solution.

4. The method according to claim 2 or 3, **characterized in that** the performic acid is in the form of an equilibrium solution.

5. The method according to claim 3, **characterized in that** said solution contains between 3 and 25, preferably 5 and 20 percent by weight of performic acid.

6. The method according to any of claims 2 - 5, **characterized in that** said fermentation process is for the production of ethanol.

7. The method according to any one of claims 2 to 6, **characterized in that** the disinfectant comprising performic acid is added to the sugar-containing material before the latter is introduced into the fermentation system.

8. The method according to any one of claims 2 to 6, **characterized in that** the disinfectant comprising performic acid is added to the sugar-containing material during the time in which that material is added to the fermentation vessel(s).

9. The method according to any one of claim 2 to 6, **characterized in that** the disinfectant comprising performic acid is added to the fermentation vessel(s) as a separate flow stream.

10. The method according to any one of claims 2 to 6 **characterized in that** the disinfectant comprising performic acid is added to the fermentation vessel(s) together with the flow stream of a sugar-containing material to be fermented, comprising molasse and/or sugar-containing juice.

11. The method according to any one of claims 2 to 6, **characterized in that** the disinfectant comprising performic acid is added to the yeast propagation tank.

12. The method according to any one of claims 2 to 6, **characterized in that** the disinfectant comprising performic acid is added to the fermentation vessel(s) together with the inoculant, such as yeast.

13. The method according to claim 2, **characterized in that** performic acid is added to the sugar-containing material in an amount of 1 - 2000 mg of performic acid per liter of sugar-containing material in the fermentation vessel(s).

14. The method according to claim 13, **characterized in that** performic acid is added to the sugar-containing material in an amount of 2 - 400, preferably 5- 100 mg of performic acid per liter of sugar-containing material in the fermentation vessel(s).

## Patentansprüche

1. Verfahren zur Prävention von bakteriellen Infektionen in einem Prozess zur Fermentation eines zuckerhaltigen Materials, **dadurch gekennzeichnet, dass** ein Perameisensäure umfassendes Desinfektionsmittel verwendet wird.

2. Verfahren nach Anspruch 1, das die Verwendung eines Fermentationssystems einschließt, welches das zuckerhaltige Material und ein Impfmittel wie Hefe umfasst, wobei das Desinfektionsmittel zum zuckerhaltigen Material und/oder zum Impfmittel gegeben wird.

3. Verfahren nach Anspruch 2, **dadurch gekennzeichnet, dass** das Desinfektionsmittel Perameisensäure in Form einer wässrigen Lösung umfasst.

4. Verfahren nach Anspruch 2 oder 3, **dadurch gekennzeichnet, dass** die Perameisensäure in Form einer Gleichgewichtslösung vorliegt.

5. Verfahren nach Anspruch 3, **dadurch gekennzeichnet, dass** die Lösung zwischen 3 und 25, vorzugsweise 5 und 20 Gew.-% Perameisensäure enthält.

6. Verfahren nach einem der Ansprüche 2 - 5, **dadurch gekennzeichnet, dass** der Fermentationsprozess zur Herstellung von Ethanol dient.

7. Verfahren nach einem der Ansprüche 2 bis 6, **dadurch gekennzeichnet, dass** das Perameisensäure umfassende Desinfektionsmittel zum zuckerhaltigen Material gegeben wird, bevor letzteres in das Fermentationssystem eingeführt wird.

8. Verfahren nach einem der Ansprüche 2 bis 6, **dadurch gekennzeichnet, dass** das Perameisensäure umfassende Desinfektionsmittel zum zuckerhaltigen Material gegeben wird, während dieses Material zu dem (den) Fermentationsbehälter(n) gegeben wird.

9. Verfahren nach einem der Ansprüche 2 bis 6, **dadurch gekennzeichnet, dass** das Perameisensäure umfassende Desinfektionsmittel als getrennter Fließstrom zu dem (den) Fermentationsbehälter(n) gegeben wird.

10. Verfahren nach einem der Ansprüche 2 bis 6, **dadurch gekennzeichnet, dass** das Perameisensäure umfassende Desinfektionsmittel zusammen mit dem Fließstrom eines zu fermentierenden zuckerhaltigen Materials, das Melasse und/oder zuckerhaltigen Saft umfasst, zu dem (den) Fermentationsbehälter(n) gegeben wird.

11. Verfahren nach einem der Ansprüche 2 bis 6, **dadurch gekennzeichnet, dass** das Perameisensäure umfassende Desinfektionsmittel zum Hefevermehrungstank gegeben wird.

12. Verfahren nach einem der Ansprüche 2 bis 6, **dadurch gekennzeichnet, dass** das Perameisensäure umfassende Desinfektionsmittel zusammen mit dem Impfmittel, wie Hefe, zu dem (den) Fermentationsbehälter(n) gegeben wird.

13. Verfahren nach Anspruch 2, **dadurch gekennzeichnet, dass** Perameisensäure in einer Menge von 1 - 2000 mg Perameisensäure pro Liter des zuckerhaltigen Materials in dem (den) Fermentationsbehälter(n) zum zuckerhaltigen Material gegeben wird.

14. Verfahren nach Anspruch 13, **dadurch gekennzeichnet, dass** Perameisensäure in einer Menge von 2 - 400, vorzugsweise 5 - 100 mg Perameisensäure pro Liter des zuckerhaltigen Materials in dem (den) Fermentationsbehälter(n) zum zuckerhaltigen Material gegeben wird.

## Revendications

1. Méthode destinée à la prévention d'une infection bactérienne dans un procédé de fermentation d'un matériau contenant des sucres, **caractérisée en ce qu'**on utilise un désinfectant comprenant de l'acide performique.

2. Méthode selon la revendication 1, comportant l'utilisation d'un système de fermentation comprenant le matériau contenant des sucres et un inoculant tel que de la levure, où le désinfectant est ajouté au matériau contenant des sucres et/ou à l'inoculant.

3. Méthode selon la revendication 2, **caractérisée en ce que** le désinfectant comprend de l'acide performique sous la forme d'une solution aqueuse.

4. Méthode selon la revendication 2 ou 3, **caractérisée en ce que** l'acide performique se trouve sous la forme d'une solution à l'équilibre.

5. Méthode selon la revendication 3, **caractérisée en ce que** ladite solution contient entre 3 et 25, préférablement entre 5 et 20% en poids, d'acide performique.

6. Méthode selon l'une quelconque des revendications 2-5, **caractérisée en ce que** ledit procédé de fermentation est destiné à la production d'éthanol.

7. Méthode selon l'une quelconque des revendications 2 à 6, **caractérisée en ce que** le désinfectant comprenant de l'acide performique est ajouté au matériau contenant des sucres avant que ce dernier ne soit introduit dans le système de fermentation.

8. Méthode selon l'une quelconque des revendications 2 à 6, **caractérisée en ce que** le désinfectant comprenant de l'acide performique est ajouté au matériau contenant des sucres lors de la période pendant laquelle ce matériau est ajouté au(x) récipient(s) de fermentation.

9. Méthode selon l'une quelconque des revendications 2 à 6, **caractérisée en ce que** le désinfectant comprenant de l'acide performique est ajouté au(x) récipient(s) de fermentation sous forme de flux d'écoulement séparé.

10. Méthode selon l'une quelconque des revendications 2 à 6, **caractérisée en ce que** le désinfectant comprenant de l'acide performique est ajouté au(x) récipient(s) de fermentation conjointement avec le flux d'écoulement d'un matériau contenant des sucres devant être fermenté, comprenant de la mélasse et/ou un jus contenant des sucres.

11. Méthode selon l'une quelconque des revendications 2 à 6, **caractérisée en ce que** le désinfectant comprenant de l'acide performique est ajouté à la cuve de propagation de levure.

12. Méthode selon l'une quelconque des revendications 2 à 6, **caractérisée en ce que** le désinfectant comprenant de l'acide performique est ajouté au(x) récipient(s) de fermentation conjointement avec l'inoculant, tel que de la levure.

13. Méthode selon la revendication 2, **caractérisée en ce que** l'acide performique est ajouté au matériau contenant des sucres selon une quantité de 1-2000 mg d'acide performique par litre de matériau contenant des sucres dans le ou les récipients de fermentation.

14. Méthode selon la revendication 13, **caractérisée en ce que** l'acide performique est ajouté au matériau contenant des sucres selon une quantité de 2-400, préférablement de 5-100 mg d'acide performique par litre de matériau contenant des sucres dans le ou les récipients de fermentation.
